# EUROPEAN PATENT APPLICATION

(11) **EP 4 736 886 A1**
(43) Date of publication of application: **06.05.2026**
(21) Application number: 24844674.2
(22) Date of filing: 17.07.2024
(51) Int. Cl.: A61K 47/68, A61K 45/00, A61K 38/07, A61K 31/537, A61P 35/00, C07K 16/00

(54) **METHOD FOR PREPARING ANTIBODY-SMALL MOLECULE DRUG CONJUGATE AND USE THEREOF**

(30) Priority: 27.07.2023 CN 202310929139
(71) Applicant: SHANGHAI RUOTUO BIOSCIENCES CO., LTD., Shanghai 201315 (CN)
(72) Inventor: HE, Honglin, Shanghai 201315 (CN); XU, Yanghua, Shanghai 201315 (CN); YE, Li, Shanghai 201315 (CN); FENG, Mengting, Shanghai 201315 (CN); XIA, Aikun, Shanghai 201315 (CN); ZHONG, Ziyang, Shanghai 201315 (CN)
(74) Representative: Canzler & Bergmeier Patentanwälte Partnerschaft mbB
(86) International application number: PCT/CN2024/105863
(87) International publication number: WO 2025/020982

(57) **Abstract**

A method for preparing an antigen binding fragment-small molecule drug conjugate and the use thereof. A method for modifying human IgG Fc comprises modifying the N-terminal sequence of human IgG Fc. When fusing the obtained IgG Fc variant and an antigen binding fragment and expressing same, the loading capacity of the functional molecule is remarkably increased, and the polymer generation following expression is greatly reduced. Also provided are a fusion protein comprising the IgG Fc variant, and a drug conjugate.

## Description

### CROSS REFERENCE TO RELATED APPLICATION

The present disclosure is an application claiming the benefit of priority to a Chinese Patent Application No. CN202310929139.3, filed on July 27, 2023, the disclosures of which are incorporated herein by reference in their entireties.

### FIELD OF TECHNOLOGY

The present disclosure relates to the technical field of biotechnology and pharmacology, and in particular to a method for preparing an antibody-drug conjugate and a use thereof.

### BACKGROUND

Antibody-drug conjugates (ADCs) are formed by chemically conjugating antibodies to small-molecule drugs. By leveraging the targeting specificity of antibodies, small-molecule drugs can be delivered to target cells to exert therapeutic effects. Owing to the combined advantages of the targeting specificity of antibodies and the high cytotoxic activity of small-molecule drugs in tumor tissues, ADCs can efficiently kill tumor cells, exhibit fewer side effects than conventional chemotherapeutic agents, and provide improved efficacy compared to traditional antibody-based anticancer drugs. As a result, ADCs are often referred to as "biological missiles" in the field of cancer therapy, demonstrating significant clinical value and becoming a major research focus in current oncology treatment.

At present, most ADC conjugation processes involve conjugating small-molecule drugs to amino acid residues in antibodies in a random or site-specific manner, with random conjugation being predominant. Random conjugation approaches include, for example, lysine residue conjugation and cysteine residue conjugation. Lysine residue conjugation utilizes the lysine residues in antibodies. An IgG molecule contains more than 80 lysine residues, among which more than 20 sites exhibit high solvent accessibility. These lysine residues can be conjugated to small-molecule drugs via acylation reactions. However, due to the large number of potential conjugation sites, this approach readily results in substantial product heterogeneity and batch-to-batch variability of ADCs, uneven distribution of the drug-to-antibody ratio (DAR), and consequently affects the study of pharmacokinetics and metabolism of ADCs. Cysteine residue conjugation utilizes the cysteine residues in antibodies. However, antibodies generally do not contain free cysteine residues available for conjugation on their surface, as the cysteine residues typically exist in the form of disulfide bonds.

Human IgG can be classified into four subclasses, namely IgG1, IgG2, IgG3, and IgG4, which differ in the positions of interchain disulfide bonds and molecular weights in their sequences. The Fc fragments of different IgG subclasses also differ in structure and function. The vast majority of ADCs that have been approved or are under development are based on conventional IgG antibodies, and among the IgG subclasses, human IgG1 is most commonly selected.

Heavy-chain antibodies were initially discovered in animals such as camels and sharks, and are novel antibody molecules that naturally lack light chains and consist only of heavy chains. Compared to conventional antibodies, heavy-chain antibodies, despite lacking light chains, retain antigen-binding capability and also possess advantages such as smaller molecular weight, high expression levels, ease of engineering modification, and good stability. Consequently, heavy-chain antibodies are increasingly being applied in the development of antibody drugs. However, to date, there have been no reports of successfully marketed ADCs prepared using heavy-chain antibodies.

When preparing ADCs using heavy-chain antibodies, small-molecule drugs are conjugated to antibodies via cysteine residues. If a human IgG 1 Fc is employed, the available interchain disulfide bonds are extremely limited. In the art, a single ADC molecule can generally contain at most four conjugated small-molecule drugs. If an enhanced therapeutic effect is desired, a greater number of conjugated small-molecule drugs is required, for example, six to eight small-molecule drugs.

Accordingly, there is an urgent need in the art to further optimize and modify ADCs to increase the number of small-molecule drugs carried per antibody and thereby improve therapeutic effect.

### SUMMARY

The present disclosure provides a method for preparing an antibody-drug conjugate and a use thereof.

In the first aspect of the present disclosure, a method for increasing the functional molecular payload of an antigen binding fragment-human IgG Fc fusion protein and reducing post-expression aggregate formation is provided, including: (1) providing an antigen binding fragment-human IgG Fc fusion protein, wherein the fusion protein includes a human IgG Fc including an engineered N-terminal sequence, wherein a method for preparing the engineered N-terminal sequence includes: replacing 6-15 amino acid residues (preferably 7-13 residues, more preferably 8-12 residues) at the N-terminus of the wild-type human IgG Fc with amino acid residues set forth in SEQ ID NO: 6 (ERKCCVECPPCP), and performing one or more of the following modifications on the amino acid sequence set forth in SEQ ID NO: 6: (a) inserting 1-9 amino acid residues between, upstream (N-terminal) of, or downstream (C-terminal) of the two cysteine residues (CC) at positions 4 and 5 of the amino acid sequence set forth in SEQ ID NO: 6; and (b) mutating one of the two cysteine residues at positions 4 and 5 of the amino acid sequence set forth in SEQ ID NO: 6.

In one or more embodiments, in (a), 2-8 non-cysteine amino acid residues (preferably 2-7 residues, more preferably 2-4 residues) are inserted between the two cysteine residues at positions 4 and 5 of the amino acid sequence set forth in SEQ ID NO: 6.

In one or more embodiments, the inserting or mutating is performed using amino acid residues selected from a group consisting of: aspartic acid, glutamic acid, alanine, glycine, valine, leucine, isoleucine, methionine, proline, tryptophan, serine, tyrosine, phenylalanine, asparagine, glutamine, threonine, lysine, arginine, and histidine.

In one or more embodiments, the inserting is performed using acidic amino acid residues, wherein the acidic amino acid residues are aspartic acid and/or glutamic acid.

In one or more embodiments, the method for increasing the functional molecular payload of the antigen binding fragment-human IgG Fc fusion protein and reducing post-expression aggregate formation further includes: (2) conjugating a functional molecule to the antigen binding fragment-human IgG Fc fusion protein, and a payload of the functional molecule is in a range of 6-8.

In one or more embodiments, the functional molecule includes: a small molecule antitumor drug, a cytotoxin, a radioisotope, a bioactive protein, a molecule targeting a tumor surface marker, a tumor-inhibitory molecule, a molecule targeting a surface marker of an immune cell, a detectable label, or an extracellular hinge region, a transmembrane domain, and an intracellular signaling domain based on chimeric antigen receptor technology, or a combination thereof.

In one or more embodiments, the molecule targeting the tumor surface marker is an antibody that binds the tumor surface marker or a ligand that binds the tumor surface marker.

In one or more embodiments, the tumor-inhibitory molecule is an antitumor cytokine or an antitumor toxin.

In one or more embodiments, the detectable label includes a fluorescent label or a chromogenic label.

In one or more embodiments, the antitumor toxin includes a toxin acting on tubulin, a toxin acting on DNA, or a compound acting on intracellular metabolism, transcription, translation, or signal transduction.

In one or more embodiments, the antitumor cytokine includes IL-2, IL-12, IL-15, IFN-beta, TNF-alpha, or variants thereof.

In one or more embodiments, the antibody that binds the tumor surface marker is an antibody that recognizes a tumor antigen.

In one or more embodiments, the toxin acting on tubulin is monomethyl auristatin, a related compound thereof, or a derivative thereof.

In one or more embodiments, the toxin acting on tubulin is a maytansinoid, a related compound thereof, or a derivative thereof.

In one or more embodiments, the toxin acting on DNA is duocarmycin, calicheamicin, pyrrolobenzodiazepines, SN-38, DXd, a related compound thereof, or a derivative thereof.

In one or more embodiments, the antitumor toxin is linked to the fusion protein via a linker, wherein the linker includes: a non-cleavable linker; a cleavable linker, including a disulfide bond-based linker, a cathepsin-sensitive linker, a hydrazone bond-based linker, or a lysosomal protease-sensitive linker.

In one or more embodiments, the non-cleavable linker is SMCC.

In one or more embodiments, the disulfide bond-based linker is sulfo-SPDB.

In one or more embodiments, the cathepsin-sensitive linker is mc-vc-PAB.

In one or more embodiments, the lysosomal protease-sensitive linker is a maleimide-GGFG peptide linker.

In one or more embodiments, the intracellular signaling domain includes: CD3ζ chain, FcεRIγ tyrosine activation motif, and the intracellular signaling domains of co-stimulatory molecules such as CD27, CD28, CD137, CD134, MyD88, or CD40.

In one or more embodiments, the transmembrane domain includes: the transmembrane domain of CD8 or CD28.

In one or more embodiments, the human IgG Fc is from human IgG1, human IgG2, or human IgG4.

In one or more embodiments, the antigen binding fragment includes, but is not limited to, a heavy-chain antibody, a single-chain fragment variable (scFv), a single-domain antibody, a bispecific T-cell engager (BiTE) antibody, a dual-affinity re-targeting (DART) antibody, or an antigen-binding polypeptide of a fragment variable (Fv) or fragment d (Fd) antibody.

In one or more embodiments, the antigen binding fragment is a variable domain of a heavy chain of an antibody, a variable domain of a light chain of an antibody, or a combination thereof.

In another aspect of the present disclosure, an engineered human IgG Fc variant is provided. The engineered human IgG Fc variant includes an engineered N-terminal sequence, wherein the engineered N-terminal sequence is obtained by: replacing 6-15 amino acid residues (preferably 7-13 residues, more preferably 8-12 residues) at the N-terminus of the wild-type human IgG Fc with amino acid residues set forth in SEQ ID NO: 6 (ERKCCVECPPCP), and performing one or more of the following modifications on the amino acid sequence set forth in SEQ ID NO: 6: (a) inserting 1-9 amino acid residues between, upstream (N-terminal) of, or downstream (C-terminal) of the two cysteine residues (CC) at positions 4 and 5 of the amino acid sequence set forth in SEQ ID NO: 6; and (b) mutating one of the two cysteine residues at positions 4 and 5 of the amino acid sequence set forth in SEQ ID NO: 6.

In one or more embodiments, in the engineered human IgG Fc variant, 2-8 amino acid residues (preferably 2-7 residues, more preferably 2-4 residues) are inserted between the two cysteine residues at positions 4 and 5 of the amino acid sequence set forth in SEQ ID NO: 6.

In one or more embodiments, in the engineered human IgG Fc variant, the inserting or mutating is performed using amino acid residues selected from a group consisting of: aspartic acid, glutamic acid, alanine, glycine, valine, leucine, isoleucine, methionine, proline, tryptophan, serine, tyrosine, phenylalanine, asparagine, glutamine, threonine, lysine, arginine, and histidine.

In one or more embodiments, in the engineered human IgG Fc variant, the inserting is performed using acidic amino acid residues, wherein the acidic amino acid residues are aspartic acid and/or glutamic acid.

In one or more embodiments, in the engineered human IgG Fc variant, the engineered N-terminal sequence of the engineered human IgG Fc variant is obtained by: mutating the amino acid sequence set forth in SEQ ID NO: 6 to an amino acid sequence set forth in SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 12, SEQ ID NO: 24, SEQ ID NO: 25, SEQ ID NO: 13, SEQ ID NO: 14, SEQ ID NO: 15, SEQ ID NO: 16, SEQ ID NO: 17, SEQ ID NO: 10, SEQ ID NO: 9, SEQ ID NO: 8, or SEQ ID NO: 7.

In another aspect of the present disclosure, a use of the aforementioned engineered human IgG Fc variant in the preparation of an antigen binding fragment-human IgG Fc fusion protein. When a functional molecule is conjugated to the antigen binding fragment-human IgG Fc fusion protein, a payload of the functional molecule is in a range of 6-8.

In another aspect of the present disclosure, a fusion protein is provided. The fusion protein is an antigen binding fragment-human IgG Fc fusion protein, wherein the fusion protein includes a human IgG Fc, wherein the human IgG Fc is the aforementioned engineered human IgG Fc variant.

In another aspect of the present disclosure, a polynucleotide is provided. The polynucleotide encodes the aforementioned engineered human IgG Fc variant or the aforementioned fusion protein.

In another aspect of the present disclosure, an expression construct (e.g., an expression vector) is provided. The expression construct includes the aforementioned polynucleotide.

In another aspect of the present disclosure, an expression system is provided. The expression system includes the aforementioned expression construct or includes a genome into which the aforementioned polynucleotide is integrated.

In another aspect of the present disclosure, a method for preparing the aforementioned engineered human IgG Fc variant or the aforementioned fusion protein is provided. The method includes: performing expression using the aforementioned expression system under a condition suitable for expression, thereby obtaining the engineered human IgG Fc variant or the fusion protein.

In another aspect of the present disclosure, a conjugate is provided. The conjugate includes: the aforementioned fusion protein and a functional molecule conjugated to the aforementioned fusion protein. A payload of the functional molecule is in a range of 6-8.

In one or more embodiments, the functional molecule includes: a small molecule antitumor drug, a cytotoxin, a radioisotope, a bioactive protein, a molecule targeting a tumor surface marker, a tumor-inhibitory molecule, a molecule targeting a surface marker of an immune cell, a detectable label, or an extracellular hinge region, a transmembrane domain, and an intracellular signaling domain based on chimeric antigen receptor technology, or a combination thereof.

In another aspect of the present disclosure, a pharmaceutical composition or kit is provided. The pharmaceutical composition or kit includes: the aforementioned fusion protein or the aforementioned conjugate.

Other aspects of the present disclosure will be obvious to those skilled in the art in view of the disclosure herein.

### BRIEF DESCRIPTION OF DRAWINGS

FIG. 1 shows a sodium dodecyl sulfate-polyacrylamide gel electrophoresis (SDS-PAGE) analysis of heavy-chain antibodies constructed using wild-type human IgG1 Fc.
FIG. 2 shows an SDS-PAGE analysis of heavy-chain antibodies constructed using mutated IgG1 Fc.
FIG. 3 shows an SDS-PAGE analysis of heavy-chain antibodies constructed using IgG1 Fc with cysteine mutations.
FIG. 4 shows an SDS-PAGE analysis of heavy-chain antibodies constructed using IgG1 Fc in which different numbers of amino acid residues are inserted between the cysteine residues at positions 4 and 5 of an amino acid sequence of IgG1 Fc.
FIG. 5 shows an SDS-PAGE analysis of heavy-chain antibodies constructed using IgG1 Fc in which amino acid residues are inserted upstream of, between, or downstream of the cysteine residues of IgG1 Fc.
FIG. 6 shows an SDS-PAGE analysis of heavy-chain antibodies constructed using IgG1 Fc into which amino acids with different properties are inserted.
FIG. 7 shows size-exclusion chromatography (SEC) results of heavy-chain antibody-small-molecule DXd drug conjugates, in which amino acids with different properties are inserted into IgG1 Fc.
FIG. 8 shows an SDS-PAGE analysis of heavy-chain antibodies constructed using human IgG2 Fc or IgG4 Fc with mutations introduced at corresponding positions.
FIG. 9 shows SEC results of heavy-chain antibody- small-molecule DXd drug conjugates constructed using human IgG2 or IgG4 Fc with mutations introduced at corresponding positions.
FIG. 10 shows the binding ability of heavy-chain antibodies containing mutated IgG1 Fc and the corresponding antibody-drug conjugates to OVCAR-3 cells.
FIG. 11 shows the killing effect of antibody-drug conjugates prepared from heavy-chain antibodies containing mutated IgG1 Fc on OVCAR-3 cells.

### DETAILED DESCRIPTION

After extensive research and experimentation, the present disclosure has disclosed a method for modifying human IgG Fc, which includes modifying the N-terminal sequence of the human IgG Fc. When the IgG Fc variant obtained according to the present disclosure is fused with an antigen binding fragment and expressed, the payload of functional molecules is significantly increased, and the formation of aggregates after expression is greatly reduced. The present disclosure further provides a fusion protein, a drug conjugate (such as an antibody-drug conjugate), and the like, including the aforementioned IgG Fc variant.

As used herein, the terms "antigen binding fragment," "antigen binding site," or "antigen binding portion" of an antibody refer to the critical region of an antibody that determines binding to an antigen, and may be the shortest sequence region sufficient to determine the binding of the antibody to the antigen.

As used herein, the terms "heavy-chain antibody," "single-domain antibody," "domain antibody," and "nanobody" are used interchangeably.

As used herein, the terms "operatively connected (linked)" or "operably attached" refer to a condition in which certain portions of a linear DNA sequence/amino acid sequence are organically connected to and functionally coordinated with other portions of the same linear DNA sequence/amino acid sequence. For example, a promoter is operably linked to a coding sequence if it controls transcription of the coding sequence.

As used herein, the terms "comprising," "having," or "including" encompass "containing," "consisting essentially of," "consisting substantially of," and "consisting of." The terms "consisting essentially of," "consisting substantially of," and "consisting of" are subordinate concepts within the scope of "comprising," "having," or "including."

As used herein, the terms "pharmaceutically acceptable carrier," "physiologically acceptable carrier," or "biologically acceptable carrier" refer to solvents, suspending agents, excipients, or the like that are used to deliver the fusion proteins of the present disclosure to a subject in need thereof (including food or feed), are controllable in terms of toxicity and side effects, and are environmentally friendly or harmless to humans and animals. The carriers may be liquid or solid, and are preferably carriers capable of maintaining the biological activity of the fusion proteins of the present disclosure to a relatively high degree.

### IgG Fc Engineering

In the inventors' prior studies, it was found that when a fusion protein is constructed using a human IgG Fc fragment and an antigen binding fragment, a large amount of aggregates are readily formed during expression, thereby adversely affecting the quality of the fusion protein, and, at the same time, the number of functional molecules that can be loaded onto the fusion protein is limited. Through in-depth research and analysis, the IgG Fc was engineered to increase the functional molecular payload of the antigen binding fragment-human IgG Fc fusion protein and to avoid the formation of aggregates, thereby endowing IgG Fc-containing fusion proteins with improved prospects for process development.

Accordingly, the present disclosure provides a novel method for engineering IgG Fc, which includes: modifying the N-terminal sequence of the wild-type human IgG Fc, specifically, a portion of the amino acid residues at the N-terminus of the wild-type human IgG Fc is replaced with the amino acid residues set forth in SEQ ID NO: 6, and one or more of the following modifications is performed on the amino acid sequence set forth in SEQ ID NO: 6, (a) inserting 1-9 amino acid residues between, upstream (N-terminal) of, or downstream (C-terminal) of the two cysteine residues (CC) at positions 4 and 5 of the amino acid sequence set forth in SEQ ID NO: 6; and (b) mutating one of the two cysteine residues at positions 4 and 5 of the amino acid sequence set forth in SEQ ID NO: 6. Such modifications yield IgG Fc variants that can be efficiently conjugated to multiple functional molecules, and the resulting antigen binding fragment-human IgG Fc fusion proteins exhibit a substantially reduced or eliminated tendency to form aggregates during expression.

The IgG Fc variants (mutant proteins) of the present disclosure are synthetic proteins or recombinant proteins, i.e., they may be produced by chemical synthesis or generated using recombinant techniques in prokaryotic or eukaryotic hosts (e.g., bacteria, yeast, or plants). Depending on the host used in the recombinant production scheme, the IgG Fc variants of the present disclosure may include or may not include an initiating methionine residue.

The present disclosure further provides fragments (biologically active fragments), derivatives, and analogs of the IgG Fc variants. As used herein, the terms "fragment," "derivative," and "analog" refer to proteins that substantially retain the same biological function or activity as the mutant proteins exemplified in the embodiments of the present disclosure.

The fragments, derivatives, or analogs of the IgG Fc variants of the present disclosure may be prepared based on the specifically exemplified IgG Fc variants of the present disclosure, which include: (i) mutant proteins in which one or more conservative or non-conservative amino acid residues (preferably conservative amino acid residues) are substituted, wherein the substituted amino acid residues may or may not be encoded by the genetic code; (ii) mutant proteins in which one or more amino acid residues bear substituent groups; (iii) mutant proteins formed by fusing the mature IgG Fc variant with another compound (such as a compound that extends the half-life of the mutant protein, e.g., polyethylene glycol); or (iv) mutant proteins formed by fusing additional amino acid sequences (such as leader sequences, signal sequences, sequences for purification of the IgG Fc variant, proprotein sequences, or fusion proteins formed with an antigen IgG fragment) to the IgG Fc variant. However, in the amino acid sequences of such fragments, derivatives, and analogs, the key mutation sites/regions claimed by the present disclosure relative to wild-type IgG Fc (e.g., the N-terminal sequence regions shown in SEQ ID NOs: 28, 29, 30, 31, 32, 33, 34, 35, 36, 12, 24, 25, 13, 14, 15, 16, 17, 10, 9, 8, and 7) are conserved; that is, the sequence variations encompassed by such fragments, derivatives, and analogs occur at non-critical regions of the IgG Fc, and such fragments, derivatives, and analogs retain the same or substantially the same functions/activities as the IgG Fc variants exemplified in the present disclosure.

In addition, the IgG Fc variants of the present disclosure may be further modified. The modifications (which generally do not alter the primary structure) include *in vivo* or *in vitro* chemical derivatizations, such as acetylation or carboxylation. The modifications also include glycosylation, such as derivative proteins produced by glycosylation modifications during the synthesis and processing of the mutant protein or during subsequent processing steps. Such modifications may be achieved by exposing the protein to enzymes capable of glycosylation (e.g., mammalian glycosyltransferases or deglycosylases). Additional modification forms include sequences containing phosphorylated amino acid residues (e.g., phosphotyrosine, phosphoserine, or phosphothreonine). The modification forms also include derivative proteins that are modified to enhance proteolytic resistance, prolong half-life, or optimize solubility. Likewise, in the amino acid sequences of the engineered IgG Fc variants, the key mutation sites/regions claimed by the present disclosure relative to wild-type IgG Fc are conserved. The sequence variations encompassed by the fragments, derivatives, and analogs occur at non-critical regions of the IgG Fc, and the fragments, derivatives, and analogs retain the same or substantially the same functions/activities as the IgG Fc variants exemplified in the present disclosure.

The term "polynucleotide encoding a mutant protein" may refer to a polynucleotide that encodes an IgG Fc variant of the present disclosure, or a polynucleotide that further includes additional coding and/or non-coding sequences.

### Fusion Proteins

The IgG Fc variant of the present disclosure may be fused with an antigen binding fragment of an antibody to form an antigen binding fragment-human IgG Fc fusion protein.

In the novel fusion proteins of the present disclosure, the antigen binding fragment and the human IgG Fc are operably linked, exhibiting excellent compatibility and high biological activity.

In a preferred embodiment of the present disclosure, the IgG Fc is linked to the antigen binding fragment via a polypeptide linker to form a fusion protein. For example, the linker may include 3-40 amino acids.

As used herein, the term "antibody" includes full-length antibodies and any antigen binding fragment (i.e., "antigen binding portion") thereof, or single chains thereof.

The term "monoclonal antibody" or "monoclonal antibody composition" refers to a preparation of antibody molecules composed of a single molecular species. A monoclonal antibody composition exhibits a single binding specificity and affinity for a particular epitope.

The term "antigen binding fragment" refers to one or more fragments of an antibody that retain the ability to specifically bind an antigen (e.g., PD-1). It should be understood that the antigen-binding function of an antibody may be exerted by fragments of a full-length antibody. Examples of binding fragments encompassed by the term "antigen binding fragment" include: (i) Fab fragments, which are monovalent fragments composed of the VL, VH, CL, and CH1 domains; (ii) F(ab')2 fragments, which are bivalent fragments including two Fab fragments linked by disulfide bonds in the hinge region; (iii) Fd fragments composed of the VH and CH1 domains; (iv) Fv fragments composed of the VL and VH domains of a single arm of an antibody; (v) dAb fragments composed of a VH domain; and (vi) isolated complementarity-determining regions (CDRs).

The antibodies of the present disclosure include humanized antibodies. The term "humanized antibody" is intended to refer to an antibody in which CDR sequences derived from the germline of another mammalian species, such as mouse, have been grafted onto a human framework sequence. Additional framework region modifications may be introduced within the human framework sequence.

The antibodies of the present disclosure also include chimeric antibodies. The term "chimeric antibody" is intended to refer to an antibody in which the variable domain sequences are derived from one species and the constant domain sequences are derived from another species, for example, an antibody in which the variable domain sequences are derived from a mouse antibody and the constant domain sequences are derived from a human antibody.

In a more preferred embodiment of the present disclosure, in the fusion protein, the IgG Fc is linked to the variable domain of a heavy-chain antibody (VHH), thereby forming a VHH-human IgG Fc fusion protein. A "heavy-chain antibody" refers to an antibody including heavy chains, wherein the antigen-binding portion contains only heavy chain variable domains and does not contain light chains or light chain variable domains.

### Nucleic Acid Molecules and Constructs or Cells Including the Same

The present disclosure further provides isolated nucleic acids encoding the human IgG Fc variants or the fusion proteins described herein, as well as complementary strands thereof.

In addition, the present disclosure provides vectors including the nucleic acid molecules encoding the human IgG Fc variants or the fusion proteins. The vectors may further include expression regulatory sequences operably linked to the nucleic acid molecules, thereby facilitating expression of the human IgG Fc variants or the fusion proteins.

A variety of suitable nucleic acid molecules encoding the human IgG Fc variants are applicable to the present disclosure. Likewise, a variety of suitable nucleic acid molecules encoding the human IgG Fc variants or the fusion proteins are also applicable to the present disclosure. The sequences referred to in the examples below are applicable to the methods of the present disclosure. It should be understood that once the amino acid sequence of a protein is provided, a person skilled in the art can readily determine the nucleic acid molecules encoding the same.

A variety of suitable vectors may be used, including cloning and expression vectors for mammals, bacteria, fungi, and yeast, as described by Pouwels et al. in Cloning Vectors: A Laboratory Manual (latest edition, Elsevier). In preferred embodiments of the present disclosure, the vector is a vector suitable for mammalian cells.

In certain embodiments, the vector may be a viral vector, including but not limited to retroviral vectors, bacteriophage vectors, adenoviral vectors, herpes simplex virus (HSV) vectors, adeno-associated virus (AAV) vectors, or lentiviral vectors.

Expression vectors include DNA sequences encoding the human IgG Fc variants or the fusion proteins, which are linked to appropriate transcriptional and translational regulatory sequences, such as those derived from mammalian, microbial, viral, or insect genes. Regulatory sequences include transcriptional promoters, operators, enhancers, ribosome binding sites, and other suitable sequences that control the initiation and termination of transcription and translation. When regulatory sequence function is required for the IgG Fc variant or fusion protein sequence, appropriate regulatory sequences are operably linked thereto. For example, a promoter sequence is linked to the front end of the DNA sequence encoding the human IgG Fc variant or the fusion protein. The ability to replicate within a host cell is typically controlled by an origin of replication. Selectable marker genes for identifying transformants may also be included in the expression vector.

In addition, a leader sequence may be fused to the coding sequence of the human IgG Fc variant or the fusion protein, thereby enabling the translated human IgG Fc variant or fusion protein to be secreted extracellularly. Signal peptides may enhance the secretion of the chimeric polypeptide from host cells to the extracellular space. The signal peptides may be cleaved during the secretion of the polypeptide from the cell.

The present disclosure further provides an expression system (e.g., a host cell) for expressing the human IgG Fc variants or fusion proteins, wherein the expression system includes the expression vector described herein or includes a genome into which the polynucleotide of the present disclosure is exogenously integrated. Any cell suitable for expression of an expression vector may serve as a host cell. For example, the host cell may be a prokaryotic cell, such as a bacterial cell; a eukaryotic cell, such as a yeast cell or a mammalian cell. Specific examples include, but are not limited to, *Escherichia coli; Streptomyces; Salmonella typhimurium;* fungal cells such as yeast, filamentous fungi; plant cells; insect cells such as *Drosophila* S2 or SF9 cells; and animal cells such as CHO, COS, HEK293, or BOWES melanoma cells, or a combination of two or more. The method for constructing the expression system is known to those skilled in the art. For example, the methods for constructing the expression system may include, but are not limited to, the microinjection method, the gene gun method, the electroporation method, the virus-mediated transduction method, the electron bombardment method, the calcium phosphate precipitation method, or any combination thereof. In preferred embodiments of the present disclosure, the expression system is an animal cell expression system, such as CHO, NS0, BHK, HEK-293, or PER-C6 cells. In more specific embodiments, the cells are CHO cells (e.g., CHO-K1, CHO-S, CHO-DG44, ExpiCHO, or CHOZN).

Methods for producing the human IgG Fc variants or fusion proteins are also included in the present disclosure. The methods include culturing recombinant cells containing nucleic acids encoding the human IgG Fc variants or fusion proteins. The methods may include allowing the cells to express the encoded human IgG Fc variants or fusion proteins and refolding the expressed human IgG Fc variants or fusion proteins. In one embodiment, the methods may further include isolation and/or purification of the refolded human IgG Fc variants or fusion proteins. The products obtained by the methods are also encompassed by the present disclosure.

The human IgG Fc variants or fusion proteins prepared as described above may be purified to a substantially homogeneous state, for example, exhibiting a single band on SDS-PAGE electrophoresis.

### Drug Conjugates

The technical solution of the present disclosure relates to the molecular design and improvement of biological macromolecules and can be applied to the development of biological macromolecular drugs.

The fusion protein of the present disclosure may be further conjugated or coupled to other heterologous functional molecules to form conjugates/coupled products (e.g., antibody-drug conjugates (ADCs)). The heterologous functional molecules may include, but are not limited to, cytokines, detectable labels, toxins (e.g., tumor-inhibitory toxins), transcriptional activation domains, transcriptional repression domains, nucleases, deaminases, methyltransferases, demethylases, transcription release factors, etc. The detectable labels may include, but are not limited to, fluorescent labels, chromogenic labels, reporter genes, localization signals, etc. The heterologous functional domains may be linked, coupled, or conjugated to the N-terminus, C-terminus, or an internal position of the fusion protein of the present disclosure.

Detectable labels may include, but are not limited to, fluorescent labels and/or chromogenic labels, such as enzymes, cofactors, fluorescent materials, luminescent materials, bioluminescent materials, radioactive materials, positron-emitting metals, and non-radioactive paramagnetic metal ions. More than one label may be included. Labels which are used to mark antibodies for detection and/or analysis and/or diagnostic purposes depend on the particular detection/analysis/diagnostic techniques and/or methods employed, such as immunohistochemical staining of (tissue) samples and flow cytometry. Suitable labels for detection/analysis/diagnostic techniques and/or methods known in the art are well understood by those skilled in the art.

The technical solution of the present disclosure may be applied to the preparation of antibody-drug conjugates (ADCs). The fusion protein carrying the variable domain of the heavy chain of a heavy-chain antibody (VHH) of the present disclosure may be conjugated to small-molecule drugs via linkers to obtain antibody-drug conjugates that combine the potent killing ability of small-molecule drugs with the targeting specificity of monoclonal antibodies. By virtue of the extremely high specificity of antibodies, the drugs can be precisely delivered into tumor cells, thereby avoiding damage to normal cells in the body and reducing adverse effects during treatment. ADCs have become promising anticancer therapeutic drugs and represent one of the fastest-growing areas in cancer treatment.

Compared with conventional antibodies, heavy-chain antibodies (HcAbs) consist only of two heavy chains and lack light chains. Therefore, they have advantages such as smaller molecular weight, higher expression levels, and better stability. Heavy-chain antibodies may also be regarded as a type of Fc fusion protein, in which the heavy chain consists of a VHH variable domain responsible for antigen binding and a conventional Fc domain. How to engineer human IgG Fc to increase the capacity for carrying targeted drugs and reduce aggregate formation, and how to successfully apply heavy-chain antibodies containing human IgG Fc to drug development, remain critical technical challenges in the art. The present disclosure provides a novel solution to these challenges. The engineered human IgG Fc disclosed herein may be used to construct heavy-chain antibodies.

In the present disclosure, the human IgG may include human IgG1, human IgG2, or human IgG4. Heavy-chain antibodies containing human IgG2 Fc exhibit weaker antibody-dependent cellular cytotoxicity (ADCC) and complement-dependent cytotoxicity (CDC) functions than those containing human IgG1 Fc. Therefore, the human IgG2 Fc provides an additional option for heavy-chain antibodies that do not require ADCC or CDC functions. Additionally, heavy-chain antibodies containing IgG2 Fc are also prone to aggregate formation for reasons similar to those of fusion proteins. Accordingly, the modification of the heavy-chain antibodies of the present disclosure is particularly important, making process development for heavy-chain antibody drugs containing human IgG2 Fc easier.

As demonstrated by the embodiments of the present disclosure, by using the optimally engineered human IgG Fc of the present disclosure, a single ADC molecule can contain six or eight conjugated small-molecule drugs. This significantly increases the small molecule drug-carrying capacity of the ADC molecule, thereby enhancing its killing effect on target cells (e.g., tumor cells). Moreover, aggregate formation is also a challenge during the preparation of ADCs using heavy-chain antibodies containing human IgG Fc. By employing the preferred mutations of the present disclosure to reduce aggregate formation, it is easier to develop a process of heavy-chain antibodies containing human IgG Fc. The functional performance of ADCs is more advantageous than that of heavy-chain antibodies containing human IgG1 Fc.

### Pharmaceutical Compositions or Kits

The present disclosure further provides a pharmaceutical composition including: the fusion protein/conjugate/coupled product (e.g., an antibody-drug conjugate) described herein, or a polynucleotide encoding the same, or an expression vector including the polynucleotide, or an expression system (e.g., a recombinant cell) expressing the fusion protein/conjugate/coupled product; and a pharmaceutically or physiologically acceptable carrier. Unless otherwise specified, the symbol "/" herein denotes "or."

Suitable pharmaceutically acceptable carriers are well known to those skilled in the art. A comprehensive description of pharmaceutically acceptable carriers can be found in Remington's Pharmaceutical Sciences. In the composition, pharmaceutically acceptable carriers may include liquids such as water, phosphate-buffered saline, Ringer's solution, normal saline, balanced salt solutions, and cryoprotectant solution (e.g., glycerol or sorbitol). In addition, the carriers may further include auxiliary substances, such as lubricants, flow-aid agents, wetting agents, emulsifiers, pH buffering agents, and stabilizers (e.g., albumin).

In use, a safe and effective amount of the fusion protein/conjugate/coupled product (e.g., an antibody-drug conjugate) described herein, or a polynucleotide encoding the same, or an expression vector including the polynucleotide, or an expression system expressing the fusion protein/conjugate/coupled product, is administered to a mammal (e.g., a human). A safe and effective amount is generally at least about 0.001 ng/kg body weight and, in most cases, does not exceed about 10 mg/kg body weight. Of course, the specific amount/dose should also take into account factors such as the route of administration and the health status of the patient, all of which are within the skill of the attending physician.

The precise effective amount for a particular subject depends on the body weight and health status of the subject, the nature and severity of the condition, and the therapeutic agent and/or combination of therapeutic agents selected for administration. For a given condition, an effective amount can be determined by routine experimentation and can be readily determined by a clinician.

The present disclosure also provides a kit or reagent kit including: the fusion protein/conjugate/coupled product (e.g., an antibody-drug conjugate) described herein, or a polynucleotide encoding the same, or an expression vector including the polynucleotide, or an expression system expressing the fusion protein/conjugate/coupled product, or the pharmaceutical composition described above.

For ease of clinical application, the pharmaceutical composition of the present disclosure may be contained in an injectable drug delivery device (e.g., a syringe), wherein the injectable drug delivery device contains a single-dose amount of the pharmaceutical composition. The injectable drug delivery device may be included in a kit to facilitate storage and use.

The kit or reagent kit of the present disclosure may further include instructions for use, so as to enable those skilled in the art to use the same in an appropriate manner.

The present disclosure will be further described below with reference to specific examples. It should be understood that these examples are provided for illustrative purposes only and are not intended to limit the scope of the present disclosure. Experimental methods for which specific conditions are not indicated in the following examples are generally carried out under conventional conditions, such as those described in Molecular Cloning: A Laboratory Manual, 3rd Edition, by J. Sambrook et al., Science Press, or in accordance with the instructions recommended by the respective manufacturers.

### Example 1. Preparation of Heavy-Chain Antibody-Drug Conjugates

To obtain heavy-chain antibody-drug conjugates, the coding genes of the variable domains of two different heavy-chain antibodies (i.e., the coding genes of VHH1 and VHH2) were each linked to the coding gene of human IgG1 Fc. The resulting genes were constructed into the multiple cloning site of the pCGS3 (Merck) expression vector. The constructed plasmids were then transfected into ExpiCHO cells for expression. Heavy-chain antibodies were purified and obtained using Protein A and were respectively designated as Antibody 1 and Antibody 2. The amino acid sequence of the human IgG1 Fc is shown below (SEQ ID NO: 1):

DKTHTCPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKAKGQP
REPQVYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

The amino acid sequences of the variable domains of the two different heavy-chain antibodies are as follows:
VHH1 (SEQ ID NO. 2): 
MGWSLILLFLVAVATRVHSQVQLVESGGGLVQPGGSLRLSCAASGITFPVNAYGWYRQAPG
KQRDLVAIISAGGTTNYADSVKGRFTISRDNSKNTLYLQMNSLRAEDTAVYYCYLQRRIGML
RDYWGQGTQVTVSS
VHH2 (SEQ ID NO. 3):

MGWSLILLFLVAVATRVHSEVQLVESGGGLVKPGGSLRLSCAASGSTSSINAMAWYRQAPG
KQREFVAGVTSSGSIVRENYAFYVKGRFTISRDNAKNSLYLQMNSLRAEDTAVYYCNAIPPY
STWGQGTQVTVSS

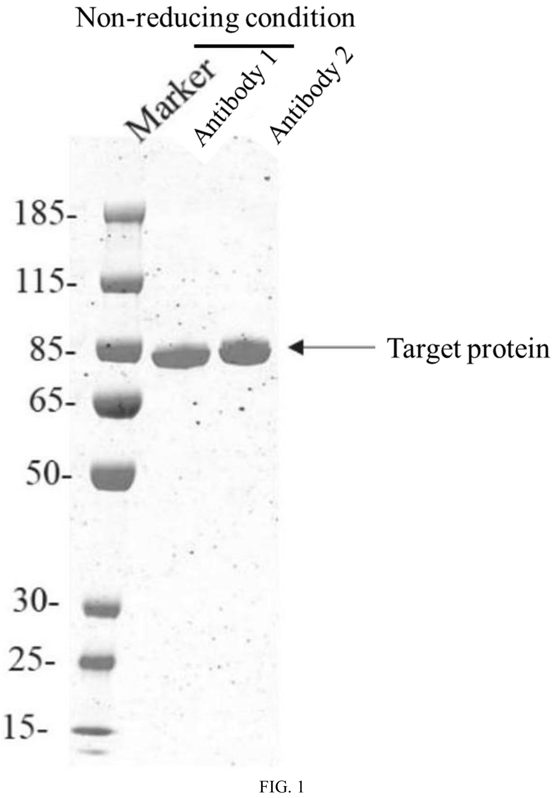

Protein purity was analyzed by SDS-PAGE. Under non-reducing conditions, the molecular weight was approximately 80 kDa. As shown in FIG. 1, under non-reducing conditions, both Antibody 1 and Antibody 2 exhibited a single band with a molecular weight of approximately 80 kDa, indicating that, after the variable domain of the heavy-chain antibody is fused with human IgG1 Fc, no aggregates were formed and the resulting antibodies exhibited high purity.

Antibody 1 and Antibody 2 were separately incubated with TCEP (Sigma) to reduce and open the disulfide bonds between the two heavy chains, followed by the addition of maleimide-GGFG-DXd (MedChemExpress) for conjugation. The conjugates were then purified using a desalting column to remove excess small-molecule drug. Analysis by hydrophobic interaction chromatography and liquid chromatography-mass spectrometry (LC-MS) demonstrated that the ratio of antibody to conjugated small-molecule drug for both Antibody 1 and Antibody 2 was 1:4.

### Example 2. Effect of Mutations on Aggregate Formation

Since the heavy-chain antibody in Example 1 could be conjugated to at most four small-molecule drugs when forming an antibody-drug conjugate, it would be more advantageous if the heavy-chain antibody could be engineered to load more small-molecule drugs. In-depth sequence analyses and experimental studies were conducted, which demonstrated that introducing mutations into a specific region of the IgG1 sequence (positions 1-10) could increase the number of disulfide bonds. The corresponding mutation sites and mutation schemes are shown in Table 1.

**Table 1**

| Name | 1 | 2 | 3 | 4 | 5 | 6 | 7 | 8 | 9 | 10 | 11 | 12 |
|---|---|---|---|---|---|---|---|---|---|---|---|---|
| IgG1 Sequence | D | K | T | H | T | C | p | P | C | P | | |
| Mutated Sequence | E | R | K | C | C | V | E | C | P | P | C | P |

The mutated sequence is ERKCCVECPPCP (SEQ ID NO: 6). The coding genes of the variable domains of two different heavy-chain antibodies VHH1 and VHH2 were respectively linked to the coding gene of the mutated IgG1 Fc.

Specifically, the gene encoding the specific region of human IgG1 Fc was first replaced accordingly to obtain the coding gene of the mutated IgG1 Fc. Then, the coding genes of the variable domains of the two different heavy-chain antibodies VHH1 and VHH2 were respectively linked to the 5' end of the coding gene of the mutated IgG1 Fc. The resulting genes were constructed into the multiple cloning site of the pCGS3 (Merck) expression vector. The constructed plasmids were transfected into ExpiCHO cells for expression, and the resulting heavy-chain antibodies were purified and obtained using Protein A. The antibodies in which VHH1 and VHH2 were respectively linked to the mutated Fc were designated as Antibody 3 and Antibody 4, respectively. Protein purity was determined by SDS-PAGE.

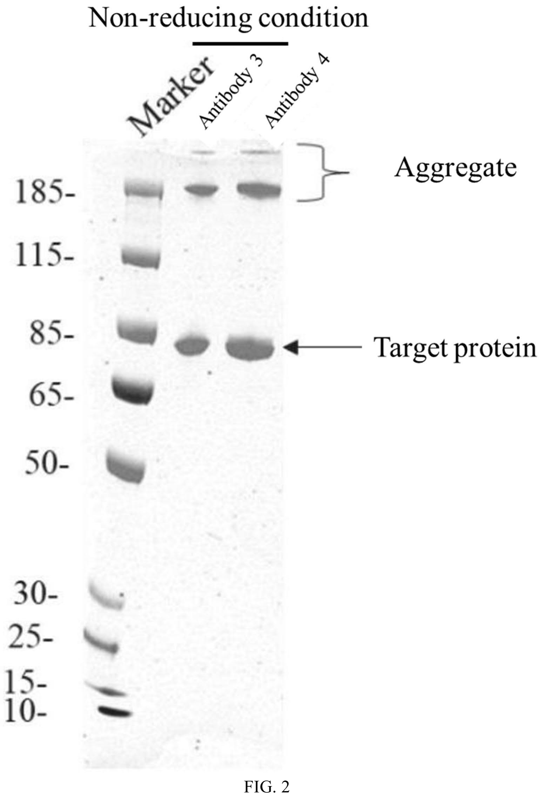

As shown in FIG. 2, under non-reducing conditions, in addition to a band corresponding to the target molecular weight, each of Antibody 3 and Antibody 4 exhibited another band corresponding to a higher molecular weight, particularly a broad band corresponding to approximately twice the target molecular weight. This indicated that after introducing the mutations into the human IgG1 Fc, the expressed heavy-chain antibodies formed a substantial amount of aggregates, especially dimers. Aggregation was observed in the two different heavy-chain antibodies (Antibody 3 and Antibody 4), indicating that this phenomenon was generally present. The aggregation may result from mispairing of interchain disulfide bonds in the mutated target region during the expression of the heavy-chain antibody. Accordingly, further optimization of the design was carried out.

Specifically, one of the cysteine residues (C) at positions 4, 5, 8, and 11 of the mutated sequence shown in Table 1 was further mutated to another amino acid residue in order to reduce the formation of one pair of disulfide bonds. The cysteine residues (C) at positions 4, 5, 8, and 11 were mutated to amino acid residues with side-chain sizes similar to those of cysteine residues. Serine (S) was used as an illustrative example, as shown in Table 2.

The specific region of the IgG1 sequence of Antibody 3 (as shown in Table 1) was mutated as shown in Table 2. The constructed plasmids were then transfected into ExpiCHO cells for expression, and the resulting heavy-chain antibodies were purified using Protein A to obtain Mutant 1, Mutant 2, Mutant 3, and Mutant 4.

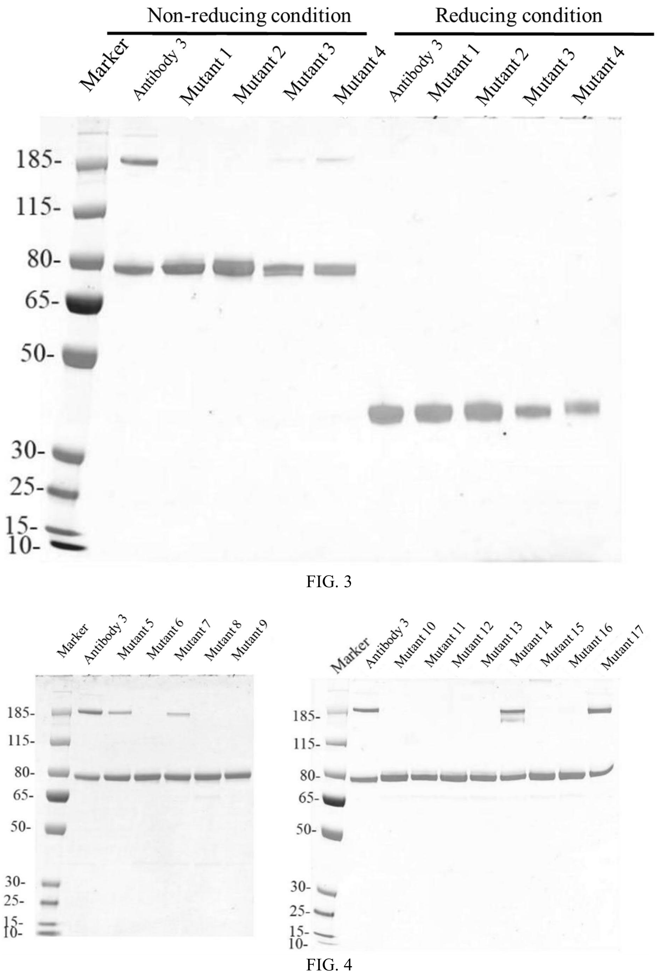

Under non-reducing conditions, the molecular weight of the mutated heavy-chain antibodies was approximately 80 kDa. As shown in FIG. 3, under non-reducing conditions, aggregate formation in Mutant 1, Mutant 2, Mutant 3, and Mutant 4 was significantly reduced, with almost no aggregates observed particularly after the mutations at positions 4 and 5.

Size-exclusion chromatography analysis was performed. The results were shown in Table 2, further demonstrating that the modification of the cysteine residues at positions 4, 5, 8, or 11 can significantly reduce aggregate formation, with the mutations at positions 4 and 5 being especially effective in reducing aggregation.

**Table 2**

| Name | Sequence | HMW (%) | MM (%) | LMW (%) |
|---|---|---|---|---|
| Antibody 3 | ERKCCVECPPCP (SEQ ID NO: 6) | 44.65 | 55.35 | 0.00 |
| Mutant 1 | ERKSCVECPPCP (SEQ ID NO: 7) | 1.13 | 98.87 | 0 |
| Mutant 2 | ERKCSVECPPCP (SEQ ID NO: 8) | 1.10 | 98.90 | 0 |
| Mutant 3 | ERKCCVESPPCP (SEQ ID NO: 9) | 4.04 | 95.96 | 0.00 |
| Mutant 4 | ERKCCVECPPSP (SEQ ID NO: 10) | 15.99 | 84.01 | 0.00 |

Analysis of expression yields showed that Antibody 1, Antibody 2, and Mutants 1-4 all exhibited expression levels in the range of 120-150 µg/mL, indicating that the introduced mutations did not adversely affect the expression levels of the heavy-chain antibodies.

### Example 3. Effect of Inserting Different Numbers of Amino Acids into the Mutated Region of a Heavy-Chain Antibody on Aggregate Formation

As can be seen from Mutants 1-4 in Example 2, the heavy-chain antibodies with the above sequence mutations not only exhibited a significant reduction in aggregate formation, but also increased the number of interchain disulfide bonds of the heavy-chain antibodies from two pairs to three pairs. Accordingly, the number of conjugated small-molecule drugs could be increased from four to six.

To further increase the number of interchain disulfide bonds of the heavy-chain antibodies, additional studies were conducted, and it was found that another modification strategy was effective, namely, inserting different numbers of amino acid residues between the cysteine residues at positions 4 and 5 of the aforementioned mutated sequence (as shown in Table 1). To evaluate the effect of inserting different numbers of amino acid residues, 1, 2, 3, 4, 5, 6, 7, 8, 9, 10, 11, 12, and 13 amino acid residues were inserted, respectively. The insertion of alanine residues (A) was used as an illustrative example, as shown in Table 3.

The coding genes of the above 13 different mutants were linked to the C-terminus of the coding gene of VHH1, respectively, followed by expression and purification to obtain Mutants 5-17.

The SDS-PAGE results were shown in FIG. 4 and Table 3. After inserting different numbers of alanine residues (A) between the cysteine residues at positions 4 and 5, aggregate formation was reduced in all mutants. In particular, the insertion of 2, 4, 5, 6, or 7 alanine residues (A) completely eliminated aggregate formation. These results indicated that inserting 2, 4, 5, 6, or 7 amino acid residues between the cysteine residues at positions 4 and 5 could substantially reduce aggregate formation of the heavy-chain antibodies.

**Table 3**

| Name | Sequence | HMW (%) | MM (%) | LMW (%) |
|---|---|---|---|---|
| Antibody 3 | ERKCCVECPPCP (SEQ ID NO: 6) | 44.65 | 55.35 | 0.00 |
| Mutant 5 | ERKCACVECPPCP (SEQ ID NO: 11) | 16.97 | 83.03 | 0.00 |
| Mutant 6 | ERKC(A)₂CVECPPCP (SEQ ID NO: 12) | 0.71 | 99.29 | 0.00 |
| Mutant 7 | ERKC(A)₃CVECPPCP (SEQ ID NO: 13) | 10.62 | 89.38 | 0.00 |
| Mutant 8 | ERKC(A)₄CVECPPCP (SEQ ID NO: 14) | 0.81 | 99.10 | 0.08 |
| Mutant 9 | ERKC(A)₃CVECPPCP (SEQ ID NO: 15) | 0.82 | 99.18 | 0.00 |
| Mutant 10 | ERKC(A)₆CVECPPCP (SEQ ID NO: 16) | 0.82 | 98.49 | 0.96 |
| Mutant 11 | ERKC(A)₇CVECPPCP (SEQ ID NO: 17) | 0.65 | 98.73 | 0.62 |
| Mutant 12 | ERKC(A)₈CVECPPCP (SEQ ID NO: 18) | 0.00 | 77.35 | 22.65 |
| Mutant 13 | ERKC(A)₉CVECPPCP (SEQ ID NO: 19) | 1.24 | 73.31 | 25.46 |
| Mutant 14 | ERKC(A)₁₀CVECPPCP (SEQ ID NO: 20) | 40.34 | 48.22 | 11.44 |
| Mutant 15 | ERKC(A)₁₁CVECPPCP (SEQ ID NO: 21) | 2.59 | 79.71 | 17.70 |
| Mutant 16 | ERKC(A)₁₂CVECPPCP (SEQ ID NO: 22) | 1.62 | 79.81 | 18.57 |
| Mutant 17 | ERKC(A)₁₃CVECPPCP (SEQ ID NO: 23) | 48.06 | 47.30 | 4.64 |

To investigate whether the insertion of amino acid residues upstream of or downstream of the cysteine residues at positions 4 and 5 could also reduce aggregate formation, two amino acid residues were inserted upstream and downstream of the cysteine residues at positions 4 and 5, respectively. The insertion of two alanine residues (A) was used as an illustrative example, as shown in Table 4.

The coding genes of IgG Fc with different mutation forms, as shown in Table 4, were linked to the 3' end of the coding gene of VHH1, respectively. The resulting genes were constructed into the pCGS3 (Merck) expression vector, and the constructed plasmids were transfected into ExpiCHO cells for expression. The heavy-chain antibodies were purified using Protein A to obtain Mutant 18 and Mutant 19.

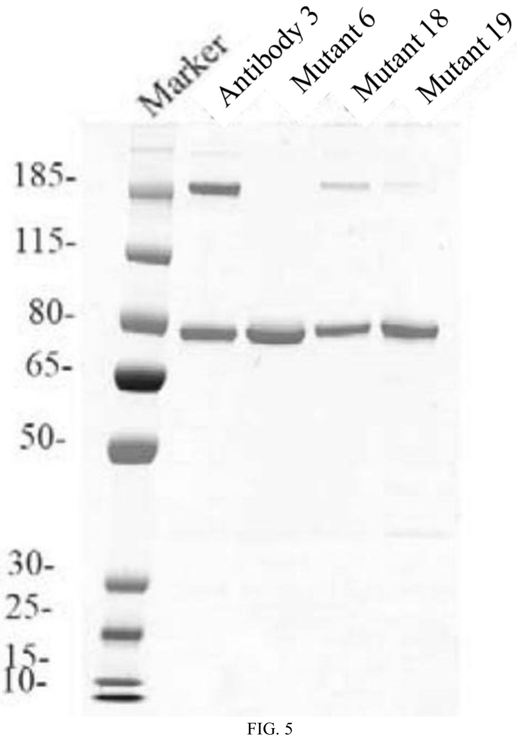

As shown in FIG. 5, compared to Antibody 3, the insertion of alanine residues (A) upstream of, between, or downstream of the cysteine residues at positions 4 and 5 (i.e., Mutant 6, Mutant 18, and Mutant 19) all resulted in reduced aggregate formation. Size-exclusion chromatography analysis was performed, and the results were shown in Table 4, indicating that the insertion of two amino acid residues between the cysteine residues at positions 4 and 5 was the most effective in reducing aggregate formation.

**Table 4**

| Name | Sequence | HMW (%) | MM (%) | LMW (%) |
|---|---|---|---|---|
| Antibody 3 | ERKCCVECPPCP (SEQ ID NO: 6) | 39.18 | 60.82 | 0.00 |
| Mutant 6 | ERKCAACVECPPCP (SEQ ID NO: 12) | 0.71 | 99.29 | 0.00 |
| Mutant 18 | ERKAACCVECPPCP (SEQ ID NO: 24) | 15.41 | 84.59 | 0.00 |
| Mutant 19 | ERKCCAAVECPPCP (SEQ ID NO: 25) | 2.89 | 97.11 | 0.00 |

The above results demonstrated that the insertion of amino acid residues upstream of, between, or downstream of the cysteine residues at positions 4 and 5 could significantly reduce aggregate formation of heavy-chain antibodies, and the insertion of amino acid residues between the cysteine residues at positions 4 and 5 provided the most pronounced effect.

### Example 4. Effect of Inserting Amino Acids with Different Properties into the Mutated Target Region on Antibody-Drug Conjugates

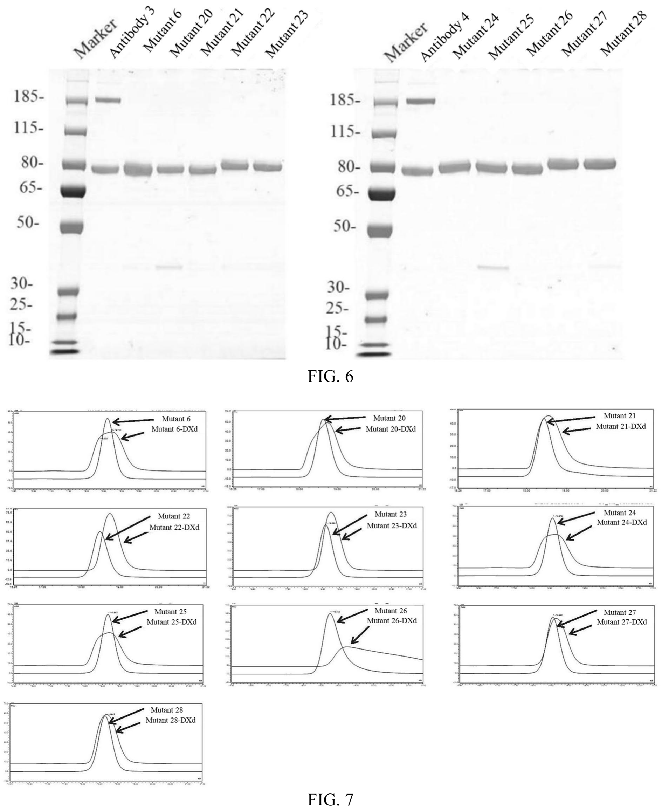

Based on Antibody 3 and Antibody 4, amino acid residues with different properties were inserted between the cysteine residues at positions 4 and 5. Specifically: nonpolar amino acids, exemplified by the insertion of two alanine residues (A); polar uncharged amino acids, exemplified by the insertion of two serine residues (S); basic amino acids, exemplified by the insertion of two lysine residues (K); and acidic amino acids, exemplified by the insertion of two glutamic acid residues (E) or two aspartic acid residues (D), as shown in Table 5. The different mutants were expressed and purified. As shown in FIG. 6, the insertion of amino acids with different properties between the cysteine residues at positions 4 and 5 significantly reduced aggregate formation compared to Antibody 3 and Antibody 4. Size-exclusion chromatography analysis further showed, as shown in Table 5, that Mutant 6 and Mutants 20-28 exhibited almost no aggregate formation.

**Table 5**

| Sequence | Name | HMW(%) | MM(%) | LMW(%) |
|---|---|---|---|---|
| ERKCCVECPPCP (SEQ ID NO: 6) | Antibody 3 | 39.18 | 60.82 | 0.00 |
| ERKCAACVECPPCP (SEQ ID NO: 24) | Mutant 6 | 0.71 | 99.29 | 0.00 |
| ERKCSSCVECPPCP (SEQ ID NO: 26) | Mutant 20 | 0.60 | 99.40 | 0.00 |
| ERKCKKCVECPPCP (SEQ ID NO: 27) | Mutant 21 | 0.00 | 99.66 | 0.34 |
| ERKCEECVECPPCP (SEQ ID NO: 28) | Mutant 22 | 0.21 | 99.79 | 0.00 |
| ERKCDDCVECPPCP (SEQ ID NO: 29) | Mutant 23 | 0.21 | 99.51 | 0.28 |
| ERKCCVECPPCP (SEQ ID NO: 6) | Antibody 4 | 39.18 | 60.82 | 0.00 |
| ERKCAACVECPPCP (SEQ ID NO: 24) | Mutant 24 | 0.00 | 100.00 | 0.00 |
| ERKCSSCVECPPCP (SEQ ID NO: 26) | Mutant 25 | 0.10 | 99.90 | 0.00 |
| ERKCKKCVECPPCP (SEQ ID NO: 27) | Mutant 26 | 0.00 | 100.00 | 0.00 |
| ERKCEECVECPPCP (SEQ ID NO: 28) | Mutant 27 | 0.23 | 99.77 | 0.00 |
| ERKCDDCVECPPCP (SEQ ID NO: 29) | Mutant 28 | 0.00 | 99.14 | 0.86 |

The above-described heavy-chain antibodies with different mutations were incubated with TCEP (Sigma) to reduce and open the interchain disulfide bonds, followed by the addition of maleimide-GGFG-DXd (MedChemExpress) for conjugation. The conjugates were then purified using a desalting column to remove excess small-molecule drug. The resulting antibody-drug conjugates were designated as Antibody 3-DXd, Mutant 6-DXd, Mutant 20-DXd, Mutant 21-DXd, Mutant 22-DXd, Mutant 23-DXd, Antibody 4-DXd, Mutant 24-DXd, Mutant 25-DXd, Mutant 26-DXd, Mutant 27-DXd, and Mutant 28-DXd.

The results showed that substantial precipitation occurred during the preparation of Antibody 3-DXd and Antibody 4-DXd. This may be caused by disulfide bond mispairing and protein structural instability during the preparation process. In contrast, almost no precipitation was observed during the preparation of the mutant-DXd conjugates, indicating a much lower probability of disulfide bond mispairing and improved protein structural stability compared to Antibody 3-DXd and Antibody 4-DXd.

Hydrophobic interaction chromatography and LC-MS analysis showed that the resulting antibody-drug conjugates had a drug-to-antibody ratio (DAR) value of 8.

Size-exclusion chromatography analysis results are shown in FIG. 7. After conjugation, Mutant 6-DXd, Mutant 20-DXd, Mutant 21-DXd, Mutant 24-DXd, Mutant 25-DXd, and Mutant 26-DXd exhibited broadened peaks with poor symmetry. This indicated that the conjugation of Mutant 6, Mutant 20, Mutant 21, Mutant 24, Mutant 25, and Mutant 26 to the small-molecule drug led to a looser molecular structure. In contrast, the peak profiles of Mutant 22-DXd, Mutant 23-DXd, Mutant 27-DXd, and Mutant 28-DXd after conjugation were nearly identical to those of corresponding mutants before conjugation, indicating that the structures of the antibody-drug conjugates derived from Mutant 22, Mutant 23, Mutant 27, and Mutant 28 did not undergo significant changes upon conjugation.

The above experiments demonstrated that the insertion of acidic amino acids, such as aspartic acid (D) or glutamic acid (E), between the cysteine residues at positions 4 and 5 not only significantly reduced aggregate formation of heavy-chain antibodies, but also enabled the prepared heavy-chain antibody-drug conjugates to maintain structural stability.

### Example 5. Effect of Inserting Different Numbers of Amino Acids into the Mutated Target Region on Antibody Stability

As can be seen from Example 4, the insertion of acidic amino acid residues, such as aspartic acid (D) or glutamic acid (E), between the cysteine residues at positions 4 and 5 not only reduced aggregation formation of heavy-chain antibodies but also facilitated the preparation of antibody-drug conjugates. Whether the insertion of greater numbers of aspartic acid (D) or glutamic acid (E) between the cysteine residues at positions 4 and 5 would affect antibody stability was investigated. With reference to Example 3, the insertion of 2, 4, 5, 6, and 7 aspartic acid (D) or glutamic acid (E), respectively, into the corresponding region of Antibody 3 was used as an illustrative example, as shown in Table 6.

**Table 6**

| Name | Sequence | HMW (%) | MM (%) | LMW (%) | Tagg (°C) |
|---|---|---|---|---|---|
| Mutant 22 | ERKCEECVECPPCP (SEQ ID NO: 28) | 0.21 | 99.79 | 0.00 | 63.28 |
| Mutant 29 | ERKCEEEECVECPPCP (SEQ ID NO: 30) | 0.43 | 99.57 | 0.00 | 61.14 |
| Mutant 30 | ERKCEEEEECVECPPCP (SEQ ID NO: 31) | 0.32 | 99.68 | 0.00 | 60.11 |
| Mutant 31 | ERKCEEEEEECVECPPCP (SEQ ID NO: 32) | 0.45 | 99.55 | 0.00 | 58.92 |
| Mutant 23 | ERKCDDCVECPPCP (SEQ ID NO: 29) | 0.21 | 99.51 | 0.28 | 63.15 |
| Mutant 33 | ERKCDDDDCVECPPCP (SEQ ID NO: 33) | 0.37 | 99.63 | 0.00 | 60.65 |
| Mutant 34 | ERKCDDDDDCVECPPCP (SEQ ID NO: 34) | 0.33 | 99.67 | 0.00 | 59.33 |
| Mutant 35 | ERKCDDDDDDCVECPPCP (SEQ ID NO: 35) | 0.35 | 99.65 | 0.00 | 56.86 |
| Mutant 36 | ERKCDDDDDDDCVECPPCP (SEQ ID NO: 36) | 0.35 | 99.65 | 0.00 | 57.66 |

The different mutants listed in Table 6 were expressed and purified. Size-exclusion chromatography analysis indicated, as shown in Table 6, that none of the heavy-chain antibodies containing these mutations exhibited aggregate formation. The thermal stability of these ten heavy-chain antibodies was further evaluated using dynamic light scattering (DLS). A DLS (Wyatt) instrument was used to monitor light scattering of the antibodies during heating from 25 °C to 85 °C, thereby determining the aggregation temperature (Tagg) of the antibodies.

As shown in Table 6, with the increase in the number of inserted aspartic acid (D) or glutamic acid (E), the aggregation temperature (Tagg) of the heavy-chain antibodies gradually decreased. Accordingly, as the number of acidic amino acids (aspartic acid (D) or glutamic acid (E)) inserted between the cysteine residues at positions 4 and 5 increased, the stability of the heavy-chain antibodies decreased. The best stability was observed when two acidic amino acids, aspartic acid (D) or glutamic acid (E), were inserted.

### Example 6. Construction of Heavy-Chain Antibodies Using Mutated Human IgG2 Fc or IgG4 Fc

To evaluate whether the above-described mutations can also be applied to human IgG2 Fc or IgG4 Fc, without adversely affecting antibody production while increasing the number of conjugated small-molecule drugs, the mutated region (exemplified by the insertion of two glutamic acid (E) or two aspartic acid (D)) of IgG1 Fc was copied to the corresponding region of IgG2 Fc and IgG4 Fc, respectively. The resulting heavy-chain antibodies were designated as Mutant 37, Mutant 38, Mutant 39, and Mutant 40, respectively, as shown in Table 7.

The amino acid sequence of human IgG2 Fc is shown below (SEQ ID NO: 4):

ERKCCVECPPCPAPPVAGPSVFLFPPKPKDTLMISRTPEVTCVVVDVSHEDPEVQFNWYVDG
VEVHNAKTKPREEQFNSTFRVVSVLTVVHQDWLNGKEYKCKVSNKGLPAPIEKTISKTKGQPREPQ
VYTLPPSREEMTKNQVSLTCLVKGFYPSDIAVEWESNGQPENNYKTTPPMLDSDGSFFLYSKLTVD
KSRWQQGNVFSCSVMHEALHNHYTQKSLSLSPGK

The amino acid sequence of human IgG4 Fc is shown below (SEQ ID NO: 5):

ESKYGPPCPSCPAPEFLGGPSVFLFPPKPKDTLMISRTPEVTCVVVDVSQEDPEVQFNWYVDG
VEVHNAKTKPREEQFNSTYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPVLDSDGSFFLYSRLTVD
KSRWQEGNVFSCSVMHEALHNHYTQKSLSLSLGK

**Table 7**

| Name | Sequence | IgG type | **HMW (%)** | MM (%) | LMW (%) |
|---|---|---|---|---|---|
| Mutant 37 | ERKCDDCVECPPCP (SEQ ID NO: 29) | IgG2 | **0.37** | 99.63 | 0.00 |
| Mutant 38 | ERKCEECVECPPCP (SEQ ID NO: 28) | IgG2 | **0.53** | 99.47 | 0.00 |
| Mutant 39 | ERKCDDCVECPPCP (SEQ ID NO: 29) | IgG4 | **0.36** | 99.64 | 0.00 |
| Mutant 40 | ERKCEECVECPPCP (SEQ ID NO: 28) | IgG4 | **0.44** | 99.56 | 0.00 |

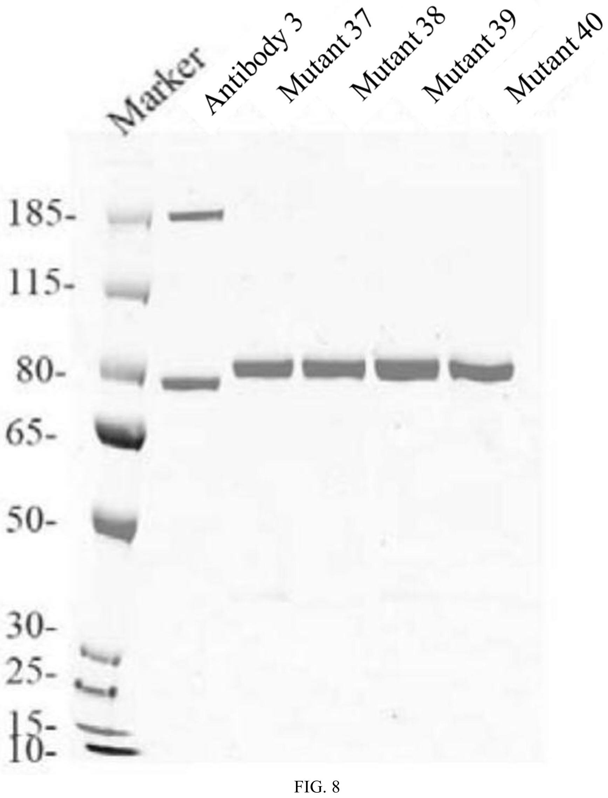

VHH1 was separately linked to the engineered human IgG2 Fc or the engineered human IgG4 Fc shown in Table 7 to construct heavy-chain antibodies. After expression and purification, Mutant 37, Mutant 38, Mutant 39, and Mutant 40 were obtained. As can be seen from the SDS-PAGE results shown in FIG. 8 and the size-exclusion chromatography analysis shown in Table 7, when the engineered human IgG2 Fc or IgG4 Fc was linked to VHH1, the obtained mutant exhibited almost no aggregate formation.

Mutant 37, Mutant 38, Mutant 39, and Mutant 40 were then used to prepare antibody-drug conjugates according to the procedure described in Example 4, yielding Mutant 37-DXd, Mutant 38-DXd, Mutant 39-DXd, and Mutant 40-DXd, respectively.

Hydrophobic interaction chromatography and LC-MS analysis showed that the drug-to-antibody ratio (DAR) values of Mutant 37-DXd, Mutant 38-DXd, Mutant 39-DXd, and Mutant 40-DXd were 8.

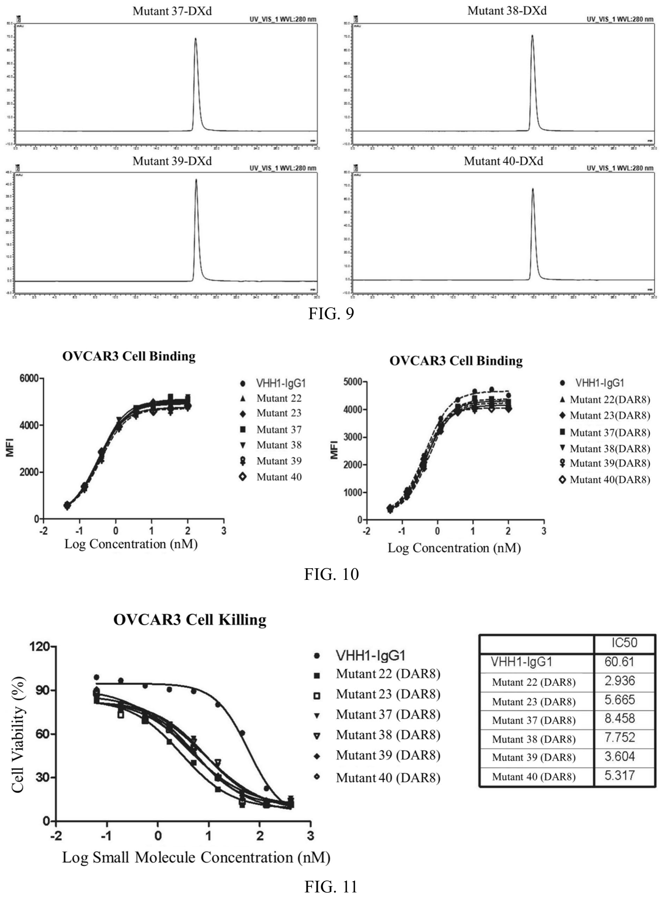

Size-exclusion chromatography analysis was performed, and the results were shown in FIG. 9. The chromatographic peak profiles of Mutant 37-DXd, Mutant 38-DXd, Mutant 39-DXd, and Mutant 40-DXd were normal, indicating that, after modification of human IgG2 or IgG4 Fc, the resulting heavy-chain antibodies not only exhibited minimal aggregate formation but also achieved an increased number of conjugated small-molecule drugs.

### Example 7. Effect of Mutated Heavy-Chain Antibodies on Cell-Binding Ability

Whether the heavy-chain antibodies with mutated Fc differ from those containing wild-type Fc in terms of cell-binding ability was further investigated.

Since VHH1 is capable of binding to the human ovarian cancer cell line OVCAR-3, flow cytometry was performed as follows: Antibody 1, Mutant 22, Mutant 23, Mutant 37, Mutant 38, Mutant 39, Mutant 40, Antibody 1-DXd, Mutant 22-DXd, Mutant 23-DXd, Mutant 37-DXd, Mutant 38-DXd, Mutant 39-DXd, and Mutant 40-DXd were first incubated with OVCAR-3 cells, unbound antibodies were washed away, followed by the addition of a PE-labeled anti-human Fc secondary antibody. After incubation, the unbound secondary antibody was washed away, and the samples were analyzed by flow cytometry. As shown in FIG. 10, the binding ability of the heavy-chain antibodies formed by VHH1 and mutated human IgG1 Fc, IgG2 Fc, or IgG4 Fc to OVCAR-3 cells was identical to that of the heavy-chain antibodies constructed using wild-type Fc. Moreover, compared to VHHI-IgG 1-DXd that only allows four conjugated small-molecule drugs, the binding ability of the mutated heavy-chain antibody-drug conjugates after loading eight small-molecule drugs to OVCAR-3 cells remained unaffected. These results demonstrated that the mutations do not impair the affinity of the heavy-chain antibodies for the antigen.

### Example 8. Preparation of Antibody-Drug Conjugates Using Mutated Heavy-Chain Antibodies and Evaluation of Their Efficacy

OVCAR-3 cells were seeded into a 96-well plate at a density of 5,000 cells per well and cultured overnight at 37°C. Antibody 1-DXd, Mutant 22-DXd, Mutant 23-DXd, Mutant 37-DXd, Mutant 38-DXd, Mutant 39-DXd, and Mutant 40-DXd were serially diluted and added to the 96-well plate containing OVCAR-3 cells, followed by incubation at 37°C for 5 days. Cell viability was measured using CellTiter-Glo (Promega) to evaluate the killing effects of the antibody-drug conjugates.

As shown in FIG. 11, compared to Antibody 1-DXd that only allows four conjugated small-molecule drugs, Mutant 22-DXd, Mutant 23-DXd, Mutant 37-DXd, Mutant 38-DXd, Mutant 39-DXd, and Mutant 40-DXd exhibited significantly enhanced killing effect on OVCAR-3 cells. When calculated based on the concentration of Dxd, the IC₅₀ values decreased by approximately 7- to 20-fold. These results indicated that antibody-drug conjugates constructed using the mutated heavy-chain antibodies were significantly more effective in killing tumor cells than those constructed using heavy-chain antibodies based on wild-type human IgG1 Fc.

The above examples only illustrate several embodiments of the present disclosure. They are described in a relatively specific and detailed manner; however, they should not be understood as a limitation on the scope of the present disclosure. It should be noted that various modifications and improvements may be made by those skilled in the art without departing from the spirit and concept of the present disclosure, and such modifications and improvements are within the protection scope of the present disclosure. Accordingly, the protection scope of the present disclosure shall be subject to the appended claims. In addition, all documents mentioned herein are incorporated by reference, as if each were individually incorporated by reference.

## Claims

1. A method for increasing payload of a functional molecule of an antigen binding fragment-human IgG Fc fusion protein and reducing post-expression aggregate formation, comprising:
(1) providing an antigen binding fragment-human IgG Fc fusion protein, wherein the fusion protein comprises a human IgG Fc comprising an engineered N-terminal sequence,
wherein a method for preparing the engineered N-terminal sequence comprises:
replacing 6-15 amino acid residues at N-terminus of wild-type human IgG Fc with amino acid residues set forth in SEQ ID NO: 6, and
performing one or more of the following modifications on the amino acid sequence set forth in SEQ ID NO: 6:
(a) inserting 1-9 amino acid residues between, upstream of, or downstream of two cysteine residues (CC) at positions 4 and 5 of the amino acid sequence set forth in SEQ ID NO: 6; and
(b) mutating one of the two cysteine residues at positions 4 and 5 of the amino acid sequence set forth in SEQ ID NO: 6.

2. The method according to claim 1, wherein in (a), 2-8 non-cysteine amino acid residues are inserted between the two cysteine residues (CC).

3. The method according to claim 1, wherein the inserting or mutating is performed using amino acid residues selected from a group consisting of: aspartic acid, glutamic acid, alanine, glycine, valine, leucine, isoleucine, methionine, proline, tryptophan, serine, tyrosine, phenylalanine, asparagine, glutamine, threonine, lysine, arginine, and histidine.

4. The method according to claim 3, wherein the inserting is performed using acidic amino acid residues, wherein the acidic amino acid residues are one or more of aspartic acid and glutamic acid.

5. The method according to claim 1, wherein the method further comprises: (2) conjugating a functional molecule to the antigen binding fragment-human IgG Fc fusion protein, wherein the payload of the functional molecule is in a range of 6-8.

6. The method according to claim 1, wherein the functional molecule comprises: a small molecule antitumor drug, a cytotoxin, a radioisotope, a bioactive protein, a molecule targeting a tumor surface marker, a tumor-inhibitory molecule, a molecule targeting a surface marker of an immune cell, a detectable label, or an extracellular hinge region, a transmembrane domain, and an intracellular signaling domain based on chimeric antigen receptor technology, or a combination thereof.

7. The method according to claim 6, wherein the molecule targeting the tumor surface marker comprises an antibody that binds the tumor surface marker or a ligand that binds the tumor surface marker; or
wherein the tumor-inhibitory molecule comprises an antitumor cytokine or an antitumor toxin; or
wherein the detectable label comprises a fluorescent label or a chromogenic label.

8. The method according to claim 7, wherein the antitumor toxin comprises a toxin acting on tubulin, a toxin acting on DNA, or a compound acting on intracellular metabolism, transcription, translation, or signal transduction; or
wherein the antitumor cytokine comprises IL-2, IL-12, IL-15, IFN-beta, TNF-alpha, or variants thereof; or
wherein the antibody that binds the tumor surface marker comprises an antibody that recognizes a tumor antigen.

9. The method according to claim 8, wherein the toxin acting on tubulin comprises monomethyl auristatin, a related compound thereof, or a derivative thereof; or
the toxin acting on tubulin comprises a maytansinoid, a related compound thereof, or a derivative thereof; or
wherein the toxin acting on DNA comprises duocarmycin, calicheamicin, pyrrolobenzodiazepines, SN-38, DXd, a related compound thereof, or a derivative thereof.

10. The method according to claim 1, wherein the human IgG Fc is from human IgG1, human IgG2, or human IgG4; or
wherein the antigen binding fragment comprises: a heavy-chain antibody, a single-chain fragment variable (scFv), a single-domain antibody, a bispecific T-cell engager (BiTE) antibody, a dual-affinity re-targeting (DART) antibody, or an antigen-binding polypeptide of a fragment variable (Fv) or fragment d (Fd) antibody; or
the antigen binding fragment comprises a variable domain of a heavy chain of an antibody, a variable domain of a light chain of an antibody, or a combination thereof.

11. An engineered human IgG Fc variant, comprising an engineered N-terminal sequence, wherein the engineered N-terminal sequence is obtained by: replacing 6-15 amino acid residues at N-terminus of wild-type human IgG Fc with amino acid residues set forth in SEQ ID NO: 6, and performing one or more of the following modifications on the amino acid sequence set forth in SEQ ID NO: 6:
(a) inserting 1-9 amino acid residues between, upstream of, or downstream of two cysteine residues (CC) at positions 4 and 5 of the amino acid sequence set forth in SEQ ID NO: 6; and
(b) mutating one of the two cysteine residues at positions 4 and 5 of the amino acid sequence set forth in SEQ ID NO: 6.

12. The engineered human IgG Fc variant according to claim 11, wherein in (a), 2-8 amino acid residues are inserted between the two cysteine residues (CC).

13. The engineered human IgG Fc variant according to claim 11, wherein the inserting or mutating is performed using amino acid residues selected from a group consisting of: aspartic acid, glutamic acid, alanine, glycine, valine, leucine, isoleucine, methionine, proline, tryptophan, serine, tyrosine, phenylalanine, asparagine, glutamine, threonine, lysine, arginine, and histidine.

14. The engineered human IgG Fc variant according to claim 11, wherein the inserting is performed using acidic amino acid residues, wherein the acidic amino acid residues are one or more of aspartic acid and glutamic acid.

15. The engineered human IgG Fc variant according to claim 11, wherein the engineered N-terminal sequence of the engineered human IgG Fc variant is obtained by: mutating the amino acid sequence set forth in SEQ ID NO: 6 to an amino acid sequence set forth in SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 12, SEQ ID NO: 24, SEQ ID NO: 25, SEQ ID NO: 13, SEQ ID NO: 14, SEQ ID NO: 15, SEQ ID NO: 16, SEQ ID NO: 17, SEQ ID NO: 10, SEQ ID NO: 9, SEQ ID NO: 8, or SEQ ID NO: 7.

16. Use of the engineered human IgG Fc variant according to any one of claims 11-15 in the preparation of an antigen binding fragment-human IgG Fc fusion protein, wherein, when a functional molecule is conjugated to the antigen binding fragment-human IgG Fc fusion protein, a payload of the functional molecule is in a range of 6-8.

17. An antigen binding fragment-human IgG Fc fusion protein, comprising a human IgG Fc, wherein the human IgG Fc is the engineered human IgG Fc variant according to any one of claims 11-15.

18. A polynucleotide, encoding the engineered human IgG Fc variant according to any one of claims 11-15 or the fusion protein according to claim 17.

19. An expression construct, comprising the polynucleotide according to claim 18.

20. An expression system, comprising the expression construct according to claim 19, or comprising a genome into which the polynucleotide according to claim 18 is integrated.

21. A method for preparing the engineered human IgG Fc variant according to any one of claims 11-15 or the fusion protein according to claim 17, comprising: performing expression using the expression system according to claim 20 under a condition suitable for expression, thereby obtaining the engineered human IgG Fc variant or the fusion protein.

22. A conjugate, comprising: the fusion protein according to claim 17 and a functional molecule conjugated to the fusion protein, wherein a payload of the functional molecule is in a range of 6-8.

23. The conjugate according to claim 22, wherein the functional molecule comprises: a small molecule antitumor drug, a cytotoxin, a radioisotope, a bioactive protein, a molecule targeting a tumor surface marker, a tumor-inhibitory molecule, a molecule targeting a surface marker of an immune cell, a detectable label, or an extracellular hinge region, a transmembrane domain, and an intracellular signaling domain based on chimeric antigen receptor technology, or a combination thereof.

24. A pharmaceutical composition or kit, comprising: the fusion protein according to claim 17, or the conjugate according to any one of claims 22-23.
